# EUROPEAN PATENT APPLICATION

(11) **EP 2 664 918 A1**
(43) Date of publication of application: **20.11.2013**
(21) Application number: 13002544.8
(22) Date of filing: 15.05.2013
(51) Int. Cl.: G01N 33/497

(54) **Breath analyzer and detachable alcohol sensor module**

(30) Priority: 18.05.2012 KR 20120052974; 14.08.2012 KR 20120089010
(71) Applicant: Sentech Korea Corporation, Gyeonggi-do 413-070 (KR)
(72) Inventor: Yoo, Do Joon, Gyeonggi-do, 463-943 (KR)
(74) Representative: Hertz, Oliver

(57) **Abstract**

A breath analyzer includes an analyzer body and an alcohol sensor module detachably attached to the analyzer body. The analyzer body includes a signal processing unit for calculating an alcohol concentration, a display unit for displaying the alcohol concentration, a connector coupling unit connected to the signal processing unit and a breath passage conduit. The alcohol sensor module includes an alcohol sensor. The alcohol sensor includes a sample gas passage and a reaction cell arranged within the sample gas passage to output an actual detection signal value corresponding to an alcohol concentration in a sample gas. The sample gas passage is composed of a sample gas inlet tube, a sample gas outlet tube and a sample gas chamber.

## Description

### Field of the Invention

The present invention relates to a breath analyzer and, more particularly, to a breath analyzer provided with a detachable alcohol sensor module.

### Background of the Invention

A breath analyzer is used in measuring the concentration of alcohol in a breath. Since the concentration of alcohol in the breath is proportional to the concentration of alcohol in the blood, the blood alcohol concentration can be calculated by measuring the concentration of alcohol in the breath.

A typical breath analyzer includes a sample gas collecting unit, a sensing unit, a signal processing unit and a display unit. The sample gas collecting unit includes a mouthpiece through which an examinee's breath is blown into the breath analyzer and a breath passage through which a sample gas introduced through the mouthpiece flows. The sensing unit includes an alcohol sensor for sensing an alcohol component existing in the sample gas collected by the sample gas collecting unit and generating an electric signal. The signal processing unit serves to analyze the electric signal supplied from the alcohol sensor and to calculate a blood alcohol concentration. The result of calculation is displayed on the display unit.

In order for the breath analyzer to accurately calculate the alcohol concentration, the breath analyzer needs to go through a calibration process prior to shipment thereof. In the calibration process, the breath analyzer is set to ensure that an electric signal indicating a standard alcohol concentration is generated when a gas having a standard alcohol concentration is supplied to a sensing unit.

For the purpose of calibration, a variable resistor has been used in the prior art. In recent years, a nonvolatile memory semiconductor such as an EEPROM or the like is used to perform calibration. The calibration is conducted in the following manner.

First, a standard alcohol gas is supplied to an alcohol sensing unit. A sensor signal generated at this time is stored in an EEPROM. Upon receiving a signal identical with the sensor signal stored in the EEPROM, a signal processing unit recognizes the alcohol concentration available at that time as a standard alcohol concentration. For more accurate calculation, standard gases having two kinds of concentration are used in some cases. In other words, if a linear equation is found from two kinds of data, it is possible to accurately calculate an alcohol concentration over a wider signal region.

However, if the breath analyzer is repeatedly used, the alcohol sensing unit is contaminated with different impurities (such as saliva and nicotine) contained in a breath. This reduces the accuracy of breath analysis, thereby necessitating recalibration. In order to perform recalibration, the breath analyzer is forwarded to an A/S center where recalibration is conducted to enhance the accuracy of breath analysis.

This poses problems in that shipping charges are incurred in the course of forwarding the breath analyzer to the A/S center and in that a user cannot use the breath analyzer during an A/S period. In case of a low-price breath analyzer, the shipping charges for recalibration in the A/S center may be higher than the price of an alcohol sensor employed in the breath analyzer. Thus, a need has existed for a solution to the problems noted above.

In view of these circumstances, the present inventor has developed breath analyzers having a detachable alcohol sensor module, which were matured into Korean Patent Nos. 10-687787 and 10-846062.

The breath analyzers disclosed in Korean Patent Nos. 10-687787 and 10-846062 are not provided with an intake pump for collecting a breath in the detachable alcohol sensor module. For that reason, there is a problem in that it is difficult to accurately analyze a breath in case of using an electrochemical alcohol sensor.

A sensor unit employing an electrochemical alcohol sensor is configured to completely decompose a specified amount of breath, eventually generating ions. The ions thus generated are moved through electrolyte. An alcohol concentration is measured using an electric current generated at this time.

Accordingly, if the collected amount of breath is changed, the alcohol concentration may be differently measured. Intake pumps for collecting a breath slightly differ in capacity from one another. If a difference in breath intake amount exists between an intake pump used in calibrating the detachable alcohol sensor module and an intake pump installed in a breath analyzer having the detachable alcohol sensor module, it is difficult to accurately measure an alcohol concentration even after calibration.

### Summary of the Invention

In view of the problems noted above, it is an object of the present invention to provide a breath analyzer which is easy to use and which is capable of preventing generation of measurement errors.

Another object of the present invention is to provide a detachable alcohol sensor module capable of being replaced with ease and capable of preventing generation of measurement errors.

In accordance with one aspect of the present invention, there is provided a breath analyzer, including:
an analyzer body including a signal processing unit for calculating an alcohol concentration, a display unit for displaying the alcohol concentration, a connector coupling unit connected to the signal processing unit and a breath passage conduit through which a user's breath passes; and
an alcohol sensor module detachably attached to the analyzer body,
wherein the alcohol sensor module includes: an alcohol sensor which includes a sample gas passage composed of a sample gas inlet tube communicating with the breath passage conduit, a sample gas outlet tube and a sample gas chamber existing between the sample gas inlet tube and the sample gas outlet tube, and a reaction cell arranged within the sample gas chamber of the sample gas passage and configured to output an actual detection signal value corresponding to an alcohol concentration in a sample gas; a nonvolatile memory unit for storing a reference detection signal value detected by the alcohol sensor when a standard gas is supplied to the alcohol sensor; a suction pump arranged to communicate with the sample gas outlet tube and configured to draw a specified amount of the sample gas into the sample gas chamber through the sample gas inlet tube; and a connector unit connected to the connector coupling unit of the analyzer body such that the actual detection signal value measured with respect to the sample gas and the reference detection signal value stored in the nonvolatile memory unit are transmitted to the signal processing unit of the analyzer body through the connector unit.

In the breath analyzer, the suction pump is one-piece formed with the alcohol sensor module. It is therefore possible to prevent generation of measurement errors otherwise caused by the variations in the characteristics of the suction pump.

The breath analyzer may further include a flow rate sensor for detecting the supply of a breath into the breath passage conduit, the breath passage conduit having a gas collection hole through which the breath is supplied to the flow rate sensor.

The breath analyzer may further include a mouthpiece slidably fitted to the breath passage conduit and kept in communication with the breath passage conduit.

In accordance with another aspect of the present invention, there is provided a detachable alcohol sensor module detachably attached to an analyzer body of a breath analyzer, including:
an alcohol sensor which includes a sample gas passage composed of a sample gas inlet tube communicating with the breath passage conduit, a sample gas outlet tube and a sample gas chamber existing between the sample gas inlet tube and the sample gas outlet tube, and a reaction cell arranged within the sample gas chamber of the sample gas passage and configured to output an actual detection signal value corresponding to an alcohol concentration in a sample gas;
a nonvolatile memory unit for storing a reference detection signal value detected by the alcohol sensor when a standard gas is supplied to the alcohol sensor;
a suction pump arranged to communicate with the sample gas outlet tube and configured to draw a specified amount of the sample gas into the sample gas chamber through the sample gas inlet tube; and
a connector unit connected to a connector coupling unit of the analyzer body such that the actual detection signal value measured with respect to the sample gas and the reference detection signal value stored in the nonvolatile memory unit are transmitted to the analyzer body through the connector unit.

The detachable alcohol sensor module may further include: a microphone installed in a through-hole formed on a side surface of the sample gas outlet tube, the microphone configured to receive, through the sample gas outlet tube, a sound wave signal generated by a breath flowing along the breath passage conduit and to generate an electric signal. The sample gas outlet tube may be made of an elastic polymer.

The volume of the sample gas introduced into the sample gas passage by the suction pump may be equal to or larger than the volume of the sample gas inlet tube and the sample gas chamber but may be smaller than the volume of the sample gas inlet tube, the sample gas chamber and a portion of the sample gas outlet tube extending between the sample gas chamber and the through-hole.

The detachable alcohol sensor module may further include: a sound wave transfer tube communicating with the sample gas outlet tube, the sound wave transfer tube configured to transfer a sound wave signal generated by a breath flowing along the breath passage conduit; and a microphone configured to receive the sound wave signal through the sound wave transfer tube and to generate an electric signal. The sound wave transfer tube may be smaller in diameter than the sample gas outlet tube.

The volume of the sample gas introduced into the sample gas passage by the suction pump may be equal to or larger than the volume of the sample gas inlet tube and the sample gas chamber but may be smaller than the volume of the sample gas inlet tube, the sample gas chamber and a portion of the sample gas outlet tube extending between the sample gas chamber and the sound wave transfer tube.

The breath analyzer according to the present invention can provide the following effects.

First, the alcohol sensor module can be easily replaced with a new one when the alcohol sensor becomes no longer usable or suffers from severe contamination. This helps enhance the ease of use of the breath analyzer.

Second, the suction pump is one-piece formed with the alcohol sensor module. Accordingly, it is possible to prevent generation of measurement errors otherwise caused by the variations in the characteristics of the suction pump.

### Brief Description of the Drawings

Fig. 1 is a perspective view showing a breath analyzer provided with a detachable alcohol sensor module according to one embodiment of the present invention.
Fig. 2 is a partial cutaway view of the breath analyzer shown in Fig. 1.
Fig. 3 is a partial exploded perspective view of the breath analyzer shown in Fig. 1.
Fig. 4 is a schematic view of a detachable alcohol sensor module employed in the breath analyzer shown in Fig. 2.
Fig. 5 is a partial exploded perspective view showing a breath analyzer provided with a detachable alcohol sensor module according to another embodiment of the present invention.
Fig. 6 is a schematic view of a detachable alcohol sensor module employed in the breath analyzer shown in Fig. 5.
Fig. 7 is a schematic view showing a detachable alcohol sensor module according to a modified embodiment.

### Detailed Description of the Preferred Embodiments

Certain embodiments of a breath analyzer and a detachable alcohol sensor module according to the present invention will now be described in detail with reference to the accompanying drawings.

The embodiments to be described later are presented by way of example in an effort to sufficiently transfer the concept of the present invention to those skilled in the relevant art. Therefore, the present invention is not limited to the following embodiments but may be embodied in many other forms. In the accompanying drawings, the width, length and thickness of the components may be illustrated on an exaggerated scale for the sake of convenience. Throughout the specification and the drawings, the same components will be designated by like reference numerals.

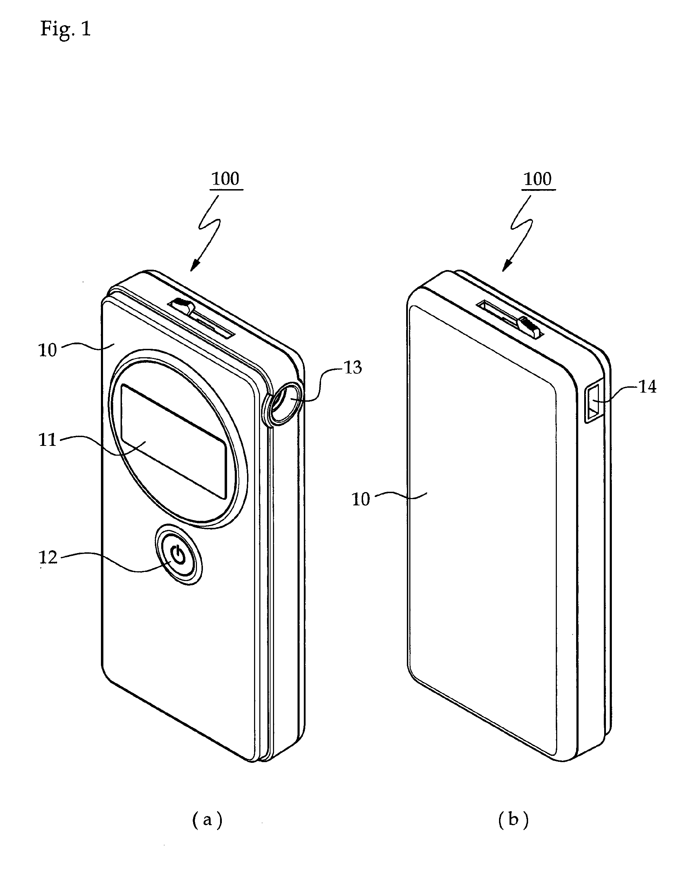

Fig. 1 is a perspective view showing a breath analyzer provided with a detachable alcohol sensor module according to one embodiment of the present invention. As shown in Fig. 1, a display unit 11 is arranged in a front upper region of a housing 10. A power switch 12 is arranged below the display unit 11. Through-holes 13 and 14 are formed in upper end regions of side surfaces of the housing 10.

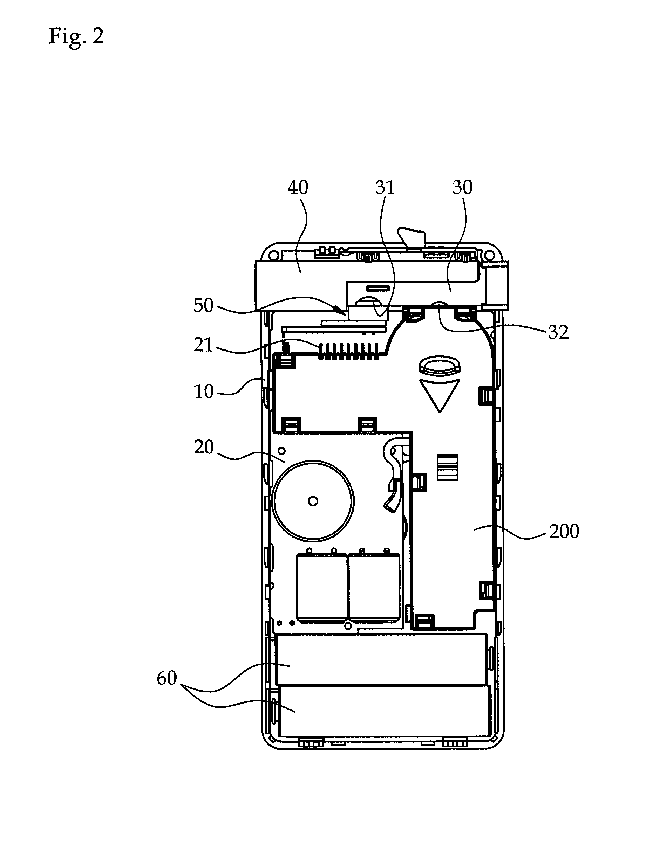

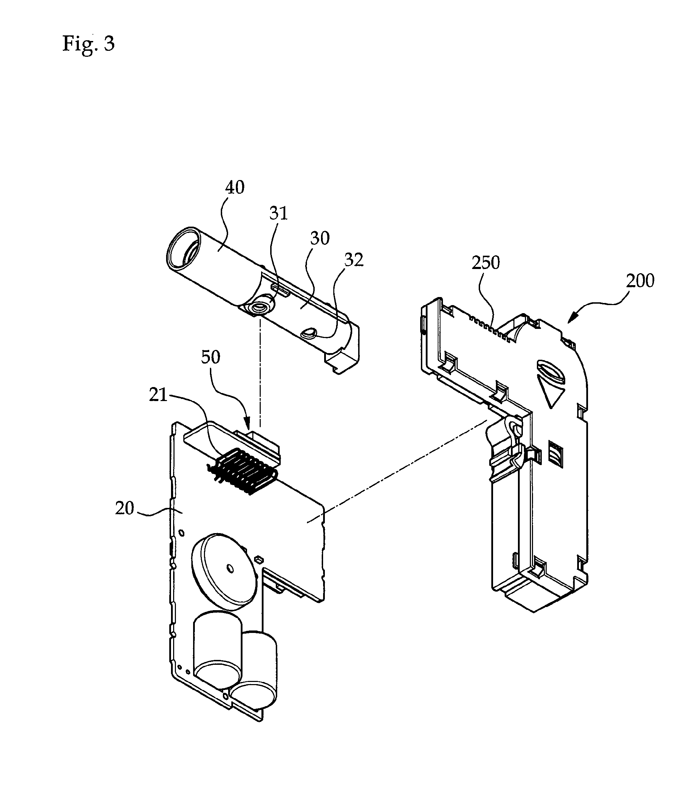

Fig. 2 is a partial cutaway view of the breath analyzer shown in Fig. 1. Fig. 3 is a partial exploded perspective view of the breath analyzer shown in Fig. 1. Referring to Figs. 1 through 3, the breath analyzer according to one embodiment of the present invention includes an analyzer body 100 and a detachable alcohol sensor module 200. The analyzer body 100 includes a housing 10 and a printed circuit board 20.

A breath passage conduit 30 through which a user's breath flows is arranged in an upper inner region of the housing 10. The breath passage conduit 30 is formed into a cylindrical shape and is opened at the opposite ends thereof. A mouthpiece 40 is slidably coupled around the breath passage conduit 30. The mouthpiece 40 is normally positioned inside the housing 10. When in use, the mouthpiece 40 is exposed to the outside through a through-hole 13 formed in an upper end region of a side surface of the housing 10. The mouthpiece 40 communicates with the breath passage conduit 30. A breath introduced through the mouthpiece 40 is moved through the breath passage conduit 30 and is discharged to the outside through a through-hole 14 formed at the opposite side from the through-hole 13. The breath passage conduit 30 has a first collection hole 31 and a second collection hole 32 through which the breath introduced into the breath passage conduit 30 is supplied to a flow rate sensor 50 and an alcohol sensor 220. The breath introduced into the breath passage conduit 30 is mostly discharged to the outside. Only a portion of the breath is supplied to the flow rate sensor 50 and the alcohol sensor 220 through the first collection hole 31 and the second collection hole 32.

The printed circuit board 20 is mounted inside the housing 10. The printed circuit board 20 is provided with a signal processing unit (not shown). The signal processing unit serves to calculate an alcohol concentration value pursuant to a detection signal generated by the detachable alcohol sensor module 200. A display unit 11 is arranged in the printed circuit board 20. The alcohol concentration value calculated in the signal processing unit is displayed on the display unit 11. A connector coupling unit 21 is arranged on one surface of the printed circuit board 20.

A flow rate sensor 50 for detecting the supply of a breath into the breath passage conduit 30 is installed at the upper end of the printed circuit board 20. The flow rate sensor 50 is arranged below the first collection hole 31 of the breath passage conduit 30. The flow rate sensor 50 detects the breath flowing out through the first collection hole 31 and, consequently, determines whether a sufficient amount of the breath is supplied into the breath passage conduit 30. Examples of the flow rate sensor 50 include a pressure sensor and a microphone. A battery 60 for supplying electric power to the printed circuit board 20 is mounted to the lower end of the housing 10.

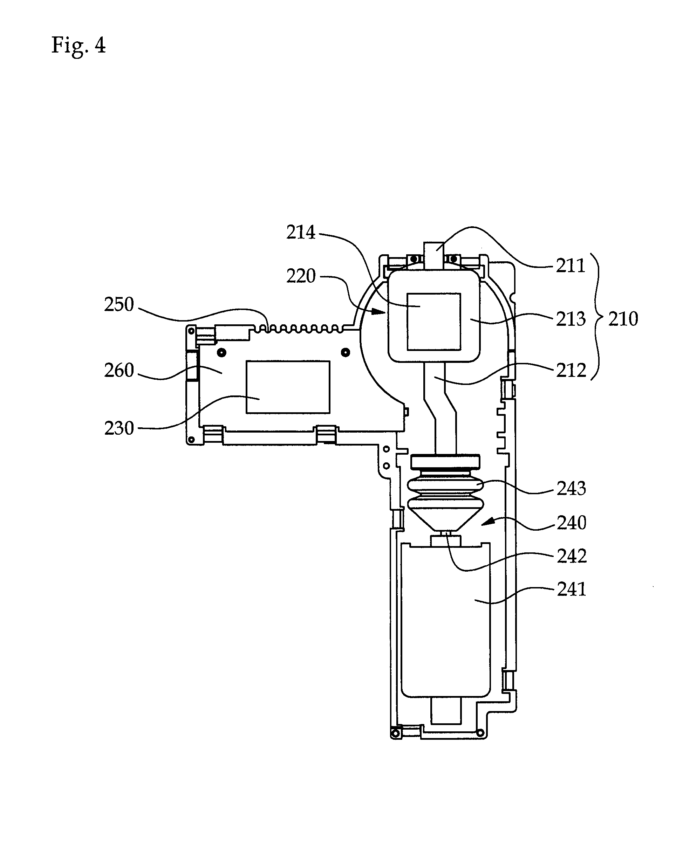

Fig. 4 is a schematic view of the detachable alcohol sensor module employed in the breath analyzer shown in Fig. 2. Referring to Fig. 4, the detachable alcohol sensor module 200 includes an alcohol sensor 220, a nonvolatile memory unit 230, a suction pump 240 and a connector unit 250.

The alcohol sensor 220 includes a sample gas passage 210 and a reaction cell 214 arranged inside the sample gas passage 210.

The sample gas passage 210 includes a sample gas inlet tube 211 through which a sample gas is introduced, a sample gas outlet tube 212 through which the sample gas is discharged and a sample gas chamber 213 for guiding the sample gas so as to flow from the sample gas inlet tube 211 toward the sample gas outlet tube 212. The breath introduced into the breath passage conduit 30 and then supplied to the sample gas passage 210 is referred to as "sample gas". The sample gas inlet tube 211 of the sample gas passage 210 is connected to the second collection hole 32 of the breath passage conduit 30. The sample gas outlet tube 212 is connected to the suction pump 240.

The reaction cell 214 is arranged in the sample gas chamber 213 existing between the sample gas inlet tube 211 and the sample gas outlet tube 212 of the sample gas passage 210. The reaction cell 214 generates an electric signal whose value is changed depending on the alcohol concentration in the sample gas.

The reaction cell 214 includes a sensing electrode, an opposite electrode and an electrolyte existing between the sensing electrode and the opposite electrode. Alcohol is decomposed into ions in the sensing electrode and is moved toward the opposite electrode through the electrolyte. An electric current is generated at this time. The value of the electric current becomes a detection signal value proportional to the alcohol concentration. The detection signal generated in the reaction cell 214 is transmitted to a printed circuit board 260 of the detachable alcohol sensor module 200.

The nonvolatile memory unit 230 may be, e.g., an Electrically Erasable Programmable Read-Only Memory (EEPROM). The nonvolatile memory unit 230 stores a reference detection signal value outputted from the alcohol sensor 220 when a standard gas having a reference alcohol concentration is used as a sample gas. The nonvolatile memory unit 230 is installed on the printed circuit board 260.

The suction pump 240 serves to draw a specified amount of the sample gas blown through the mouthpiece 40 into the sample gas passage 210 and to discharge the sample gas from the sample gas passage 210. The suction pump 240 is installed so as to communicate with the sample gas outlet tube 212 of the sample gas passage 210. The suction pump 240 is momentarily operated a specified time after a user blows a breath into the mouthpiece 40, thereby collecting a specified amount of the sample gas. The flow rate sensor 50 is used to determine whether a user starts to blow a breath into the mouthpiece 40.

A solenoid pump may be used as the suction pump 240. The suction pump 240 includes a housing 241 having a bobbin (not shown) wound with an induction coil and a plunger 242 vertically movably installed in the bore of the housing 241. The plunger 242 is connected to a bellows 243 which can be expanded and contracted.

If an electric current is instantaneously applied to the induction coil, the plunger 242 is moved backward by a specified distance and is then returned to an original position by virtue of a return spring (not shown) arranged within the housing 241. During the backward movement of the plunger 242, the bellows 243 is expanded to thereby introduce the sample gas into the sample gas passage 210. During the returning process of the plunger 242, the bellows 243 is contracted to thereby discharge the sample gas existing in the sample gas chamber 213 to the outside through the sample gas inlet tube 211. By limiting the displacement of the plunger 242, it is possible to collect a specified amount of the sample gas and to discharge a specified amount of the sample gas.

When introduced into and discharged from the sample gas passage 210, the sample gas is adsorbed to the reaction cell 214 arranged in the sample gas chamber 213. The reaction cell 214 generates an electric signal pursuant to the alcohol concentration in the sample gas.

In the present breath analyzer, the suction pump 240 is installed in the detachable alcohol sensor module 200. This makes it possible to prevent generation of errors otherwise caused by the difference in sample gas introduction amount which depends on the kind of the suction pump 240.

The connector unit 250 is installed on the printed circuit board 260 and is connected to the connector coupling unit 21 of the analyzer body 100. The reference detection signal value stored in the nonvolatile memory unit 230 and the detection signal value measured by the alcohol sensor 220 are transmitted to the signal processing unit of the analyzer body 100 via the connector unit 250. A suction pump operating signal generated by the signal processing unit and an electric current are supplied to the suction pump 240 of the detachable alcohol sensor module 200 via the connector unit 250.

Description will now be made on the operation of the breath analyzer configured as above.

First, a calibration process of the detachable alcohol sensor module 200 will be described. The detachable alcohol sensor module 200 is mounted on a calibration device. Then, a standard alcohol gas is supplied to the detachable alcohol sensor module 200. The suction pump 240 is instantaneously operated to collect a specified amount of the sample gas which in turn is decomposed into ions. The value of an electric current generated by the ions is stored in the nonvolatile memory unit 230 as a reference detection signal value.

In the present invention, the reference detection signal value stored in the nonvolatile memory unit 230 is set in view of the variations in the characteristics of the reaction cell 214 of the alcohol sensor 220 and the variations in the characteristics of the suction pump 240. Accordingly, even if the detachable alcohol sensor module 200 is attached to the analyzer body 100, it is possible to accurately measure the alcohol concentration as in the calibration process.

Next, description will be made on the replacement of the detachable alcohol sensor module 200 and the use of the breath analyzer. In case where the detachable alcohol sensor module 200 is to be replaced with a new one, the back cover of the housing 10 of the analyzer body 100 is opened and, then, the detachable alcohol sensor module 200 is removed from the connector coupling unit 21. Thereafter, the connector unit 250 of a new detachable alcohol sensor module 200 is coupled to the connector coupling unit 21. At the same time, the sample gas inlet tube 211 of the sample gas passage 210 is inserted into the second collection hole 32 of the breath passage conduit 30.

Since the reference detection signal value set in view of the variations in the characteristics of the reaction cell 214 and the variations in the characteristics of the suction pump 240 is already stored in the nonvolatile memory unit 230, it is possible to accurately measure the alcohol concentration without having to perform any additional calibration.

If a user begins to blow his or her breath into the breath analyzer through the mouthpiece 40 while pushing the power switch 12, the breath flows along the breath passage conduit 30. If the flow rate sensor 50 makes sure that a sufficient amount of the breath flows through the breath passage conduit 30, the suction pump 240 is operated after a while to introduce a part of the breath, i.e., a sample gas, into the sample gas passage 210.

The alcohol contained in the sample gas is completely decomposed into ions in the sensing electrode of the reaction cell 214. The ions thus generated are moved toward the opposite electrode through the electrolyte. The value of an electric current generated at this time becomes a detection signal value proportional to an alcohol concentration in the sample gas.

The detection signal value thus generated and the reference detection signal value stored in the nonvolatile memory unit 230 are transmitted to the signal processing unit of the analyzer body 100. The signal processing unit calculates an alcohol concentration using the detection signal values. The alcohol concentration thus calculated is displayed on the display unit 11.

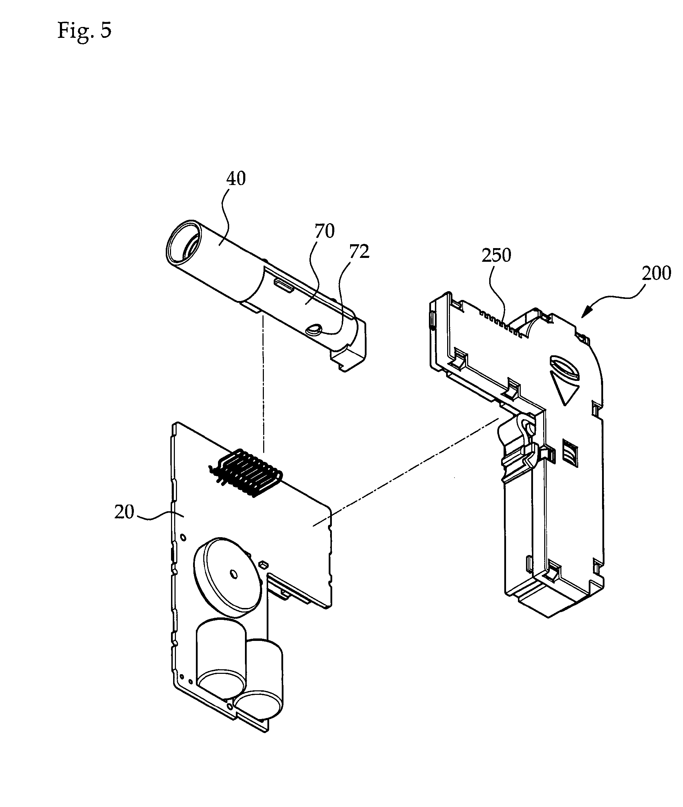

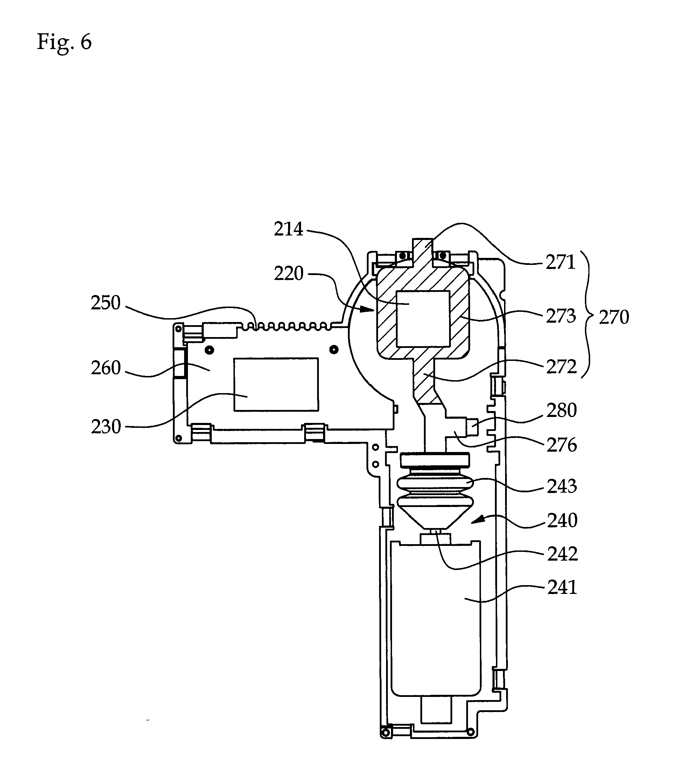

Fig. 5 is a partial exploded perspective view showing a breath analyzer provided with a detachable alcohol sensor module according to another embodiment of the present invention. Fig. 6 is a schematic view of a detachable alcohol sensor module employed in the breath analyzer shown in Fig. 5.

The breath analyzer of the present embodiment differs from the breath analyzer shown in Fig. 2 in terms of the structure for detecting the supply of a breath into the breath passage conduit 70. Only the differing point will be described in detail with no description made on the remaining points.

In the present embodiment, as shown in Fig. 5, the first collection hole is not formed in the breath passage conduit 70. Only one collection hole 72 corresponding to the second collection hole 32 of the breath analyzer shown in Fig. 2 is formed in the breath passage conduit 70. For that reason, there is no need to provide a flow rate sensor otherwise arranged below the first collection hole of the breath passage conduit 70 to determine whether a sufficient amount of the breath is supplied into the breath passage conduit 70.

As shown in Fig. 6, the detachable alcohol sensor module according to the present embodiment includes a sample gas passage 270 communicating with the breath passage conduit 70, a sound wave transfer tube 276 connected to the sample gas passage 270 and a microphone 280. The sample gas passage 270 includes a sample gas inlet tube 271, a sample gas chamber 273 and a sample gas outlet tube 272.

The sound wave transfer tube 276 is connected to the sample gas outlet tube 272 of the sample gas passage 270. The sound wave transfer tube 276 serves to transfer a sound wave signal generated by the breath flowing through the breath passage conduit 70 to the microphone 280 installed at the end of the sound wave transfer tube 276. The microphone 280 may be inserted into the sound wave transfer tube 276 which is made of an elastic polymer. The sound wave signal generated by the breath flowing through the breath passage conduit 70 is transferred to the microphone 280 via the sample gas inlet tube 271, the sample gas chamber 273, the sample gas outlet tube 272 and the sound wave transfer tube 276.

It is preferred that the sound wave transfer tube 276 is smaller in diameter than the sample gas outlet tube 272. This is because it is desirable that only the sound wave is transferred to the sound wave transfer tube 276 with the sample gas not introduced into the sound wave transfer tube 276.

As indicated by hatching lines in Fig. 6, the amount of the sample gas introduced into the sample gas passage 270 by the suction pump 240 is preferably equal to or larger than the volume of the sample gas inlet tube 271 and the sample gas chamber 273 but is preferably smaller than the volume of the sample gas inlet tube 271, the sample gas chamber 273 and the portion of the sample gas outlet tube 272 extending between the sample gas chamber 273 and the sound wave transfer tube 276. This is to prevent the sample gas from flowing into the sound wave transfer tube 276 and contaminating the microphone 280. As mentioned earlier, the amount of the sample gas introduced into the sample gas passage 270 can be adjusted by limiting the displacement of the plunger 242 of the suction pump 240.

If the amount of the sample gas introduced into the sample gas passage 270 by the suction pump 240 is set smaller than the volume of the sample gas inlet tube 271, the sample gas chamber 273 and the portion of the sample gas outlet tube 272 extending between the sample gas chamber 273 and the sound wave transfer tube 276 and if the diameter of the sound wave transfer tube 276 is set smaller than the diameter of the sample gas outlet tube 272, it is possible to prevent the sample gas from reaching the microphone 280. For the same reasons as stated above, it is preferred that the sound wave transfer tube 276 is connected to the sample gas outlet tube 272 in a position nearer to the suction pump 240.

The microphone 280 is a means for converting a sound wave signal to an electric signal. In the present embodiment, the microphone 280 converts a sound wave signal, which is generated when the breath flowing through the breath passage conduit 70 is discharged to the outside, to an electric signal. Accordingly, it is possible to confirm, through the electric signal of the microphone 280, whether a user begins to blow a breath.

In the present embodiment, the microphone 280 for determining whether a sufficient amount of the breath is supplied to the breath passage conduit 70 is installed in a position at which the sample gas is hard to arrive. It is therefore possible to prevent the microphone 280 from being contaminated with saliva or moisture contained in the breath. Accordingly, it is possible to accurately determine whether a sufficient amount of the breath flows through the breath passage conduit 70.

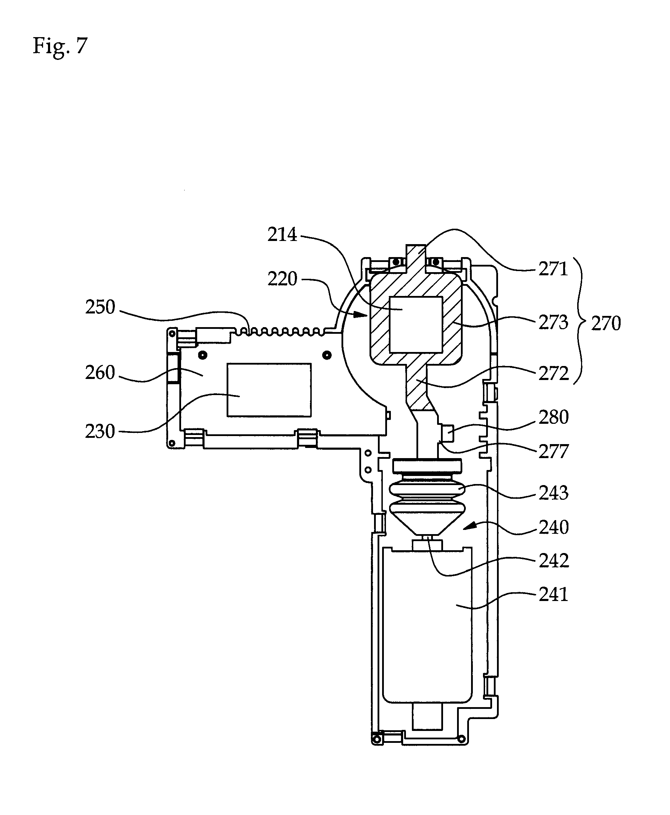

Fig. 7 is a schematic view showing a detachable alcohol sensor module according to a modified embodiment. The detachable alcohol sensor module of the present embodiment differs from the module shown in Fig. 6 in terms of the installation method of the microphone 280. Therefore, only the installation method of the microphone 280 will be described in detail. The same components will be designated by like reference symbols with no description made thereon.

In the present embodiment, a through-hole 277 is formed on a side surface of the sample gas outlet tube 272. The microphone 280 is inserted into the through-hole 277. The sample gas outlet tube 272 is preferably made of an elastic polymer. The diameter of the through-hole 277 is preferably smaller than the diameter of the microphone 280. In this case, the sample gas outlet tube 272 can make close contact with the microphone 280 due to the elasticity of the sample gas outlet tube 272, thereby eliminating a gap which may otherwise exist between the sample gas outlet tube 272 and the microphone 280. In order to enhance sealing performance, a portion of the outer circumferential surface of the sample gas outlet tube 272 existing around the through-hole 277 extends outward so as to surround the microphone 280.

While certain preferred embodiments of the invention have been described above, the scope of the present invention is not limited to these embodiments. It will be apparent to those skilled in the relevant art that various changes, modifications and substitutions may be made without departing from the scope of the invention defined in the claims. Such changes, modifications and substitutions shall be construed to fall within the scope of the present invention.

## Claims

1. A breath analyzer, comprising:
an analyzer body including a signal processing unit for calculating an alcohol concentration, a display unit for displaying the alcohol concentration, a connector coupling unit connected to the signal processing unit and a breath passage conduit through which a user's breath passes; and
an alcohol sensor module detachably attached to the analyzer body,
wherein the alcohol sensor module includes: an alcohol sensor which includes a sample gas passage composed of a sample gas inlet tube communicating with the breath passage conduit, a sample gas outlet tube and a sample gas chamber existing between the sample gas inlet tube and the sample gas outlet tube, and a reaction cell arranged within the sample gas chamber of the sample gas passage and configured to output an actual detection signal value corresponding to an alcohol concentration in a sample gas; a nonvolatile memory unit for storing a reference detection signal value detected by the alcohol sensor when a standard gas is supplied to the alcohol sensor; a suction pump arranged to communicate with the sample gas outlet tube and configured to draw a specified amount of the sample gas into the sample gas chamber through the sample gas inlet tube; and a connector unit connected to the connector coupling unit of the analyzer body such that the actual detection signal value measured with respect to the sample gas and the reference detection signal value stored in the nonvolatile memory unit are transmitted to the signal processing unit of the analyzer body through the connector unit.

2. The breath analyzer of claim 1, wherein the analyzer body further includes a flow rate sensor for detecting the supply of a breath into the breath passage conduit, the breath passage conduit having a gas collection hole through which the breath is supplied to the flow rate sensor.

3. The breath analyzer of claim 1, further comprising:
a microphone installed in a through-hole formed on a side surface of the sample gas outlet tube, the microphone configured to receive, through the sample gas outlet tube, a sound wave signal generated by a breath flowing along the breath passage conduit and to generate an electric signal.

4. The breath analyzer of claim 3, wherein the sample gas outlet tube is made of an elastic polymer.

5. The breath analyzer of claim 3, wherein the volume of the sample gas introduced into the sample gas passage by the suction pump is equal to or larger than the volume of the sample gas inlet tube and the sample gas chamber but is smaller than the volume of the sample gas inlet tube, the sample gas chamber and a portion of the sample gas outlet tube extending between the sample gas chamber and the through-hole.

6. The breath analyzer of claim 1, further comprising:
a sound wave transfer tube communicating with the sample gas outlet tube, the sound wave transfer tube configured to transfer a sound wave signal generated by a breath flowing along the breath passage conduit; and
a microphone configured to receive the sound wave signal through the sound wave transfer tube and to generate an electric signal.

7. The breath analyzer of claim 6, wherein the sound wave transfer tube is smaller in diameter than the sample gas outlet tube.

8. The breath analyzer of claim 6, wherein the volume of the sample gas introduced into the sample gas passage by the suction pump is equal to or larger than the volume of the sample gas inlet tube and the sample gas chamber but is smaller than the volume of the sample gas inlet tube, the sample gas chamber and a portion of the sample gas outlet tube extending between the sample gas chamber and the sound wave transfer tube.

9. The breath analyzer of claim 1, wherein the analyzer body further includes a mouthpiece slidably fitted to the breath passage conduit and kept in communication with the breath passage conduit.

10. A detachable alcohol sensor module detachably attached to an analyzer body of a breath analyzer, comprising:
an alcohol sensor which includes a sample gas passage composed of a sample gas inlet tube communicating with the breath passage conduit, a sample gas outlet tube and a sample gas chamber existing between the sample gas inlet tube and the sample gas outlet tube, and a reaction cell arranged within the sample gas chamber of the sample gas passage and configured to output an actual detection signal value corresponding to an alcohol concentration in a sample gas;
a nonvolatile memory unit for storing a reference detection signal value detected by the alcohol sensor when a standard gas is supplied to the alcohol sensor;
a suction pump arranged to communicate with the sample gas outlet tube and configured to draw a specified amount of the sample gas into the sample gas chamber through the sample gas inlet tube; and
a connector unit connected to a connector coupling unit of the analyzer body such that the actual detection signal value measured with respect to the sample gas and the reference detection signal value stored in the nonvolatile memory unit are transmitted to the analyzer body through the connector unit.

11. The detachable alcohol sensor module of claim 10, further comprising:
a microphone installed in a through-hole formed on a side surface of the sample gas outlet tube, the microphone configured to receive, through the sample gas outlet tube, a sound wave signal generated by a breath flowing along the breath passage conduit and to generate an electric signal.

12. The detachable alcohol sensor module of claim 11, wherein the sample gas outlet tube is made of an elastic polymer.

13. The detachable alcohol sensor module of claim 11, wherein the volume of the sample gas introduced into the sample gas passage by the suction pump is equal to or larger than the volume of the sample gas inlet tube and the sample gas chamber but is smaller than the volume of the sample gas inlet tube, the sample gas chamber and a portion of the sample gas outlet tube extending between the sample gas chamber and the through-hole.

14. The detachable alcohol sensor module of claim 10, further comprising:
a sound wave transfer tube communicating with the sample gas outlet tube, the sound wave transfer tube configured to transfer a sound wave signal generated by a breath flowing along the breath passage conduit; and
a microphone configured to receive the sound wave signal through the sound wave transfer tube and to generate an electric signal.

15. The detachable alcohol sensor module of claim 14, wherein the sound wave transfer tube is smaller in diameter than the sample gas outlet tube.

16. The detachable alcohol sensor module of claim 14, wherein the volume of the sample gas introduced into the sample gas passage by the suction pump is equal to or larger than the volume of the sample gas inlet tube and the sample gas chamber but is smaller than the volume of the sample gas inlet tube, the sample gas chamber and a portion of the sample gas outlet tube extending between the sample gas chamber and the sound wave transfer tube.
